**Europäisches Patentamt**

**European Patent Office**

**Office européen des brevets**

(11) Publication number: **0 162 610**
**B1**

(12) **EUROPEAN PATENT SPECIFICATION**

(45) Date of publication of patent specification: **15.06.88**

(21) Application number: **85302945.2**

(22) Date of filing: **26.04.85**

(51) Int. Cl.⁴: **A 61 F 13/10,** A 61 F 5/01

(54) **Improvements in or relating to wrist supports.**

(30) Priority: **02.05.84 GB 8411209**

(43) Date of publication of application:
**27.11.85 Bulletin 85/48**

(45) Publication of the grant of the patent:
**15.06.88 Bulletin 88/24**

(84) Designated Contracting States:
**BE CH DE FR GB LI NL SE**

(56) References cited:
**DE-A-3 028 381**
**DE-C- 587 234**
**FR-A-1 124 593**
**GB-A-1 093 338**
**US-A-3 327 703**
**US-A-3 533 407**
**US-A-4 315 504**

(73) Proprietor: **SPENCER (BANBURY) LIMITED**
**Spencer House Britannia Road**
**Banbury Oxfordshire OX16 8DP (GB)**

(72) Inventor: **Rice, Philip Cracroft**
**1 Brook Street Fenny Compton**
**Leamington Spa Warwickshire CV33 OYH (GB)**

(74) Representative: **Cole, Paul Gilbert et al**
**Hughes Clark Byrne & Parker 63 Lincoln's Inn**
**Fields P.O. Box 22**
**London WC2A 3JU (GB)**

Courier Press, Leamington Spa, England.

## Description

This invention relates to a wrist support.

A wrist support for use by sports people in which a metal sheet or strip in a pocket is held by semi-flexible fabric to the dorsal surface of the wrist is described in Patent Specification No. US—A—3533407. But the support does not hold the hand in dorsoflexion as is required in the treatment of more serious wrist injuries and rehabilitation.

Patent Specification No. US—A—3327703 describes and claims a wrist support adapted to extend along and surround the forearm, wrist and palm of the hand to restrict mobility of the wrist joint, the support being formed from stretch fabric comprising a combination of more and less easily stretchable transverse bands, the support being formed in its outer surface with a longitudinal pocket for a thin brace and having a cut-out defining a thumb aperture positioned relative to the longitudinal pocket so that when in place the pocket extends along the ventral surface of the forearm and wrist and underlies the palm, and the band of less stretchable material being positioned towards the proximal end of the support so as to act on the wrist but substantially not on the palm. However, the support is made from fabric folded over and joined edge to edge by stitching to form a tube; it further comprises proximal and distal portions sewn together.

This invention provides a wrist support as aforesaid characterised in that it is formed from a single piece of fabric consisting of a single less easily stretchable band between proximal and distal more easily stretchable bands and in that the support has lateral edges with attachment means thereat for fastening the lateral edges together about the wrist.

The use of a single piece of stretch fabric having purpose-designed asymmetrically located bands of different elastication is believed novel and leads to significant simplification of the manufacturing process, again without jeopardising the fit of the support or its function in supporting the wrist. When it is not being worn, the fabric of the support is undistorted which gives the support a more professionally-made and attractive appearance to the user without jeopardising the support provided. The effect of the band positioning as aforesaid is to concentrate the support where it is needed without exerting unwanted pressure across the palm of the hand.

The invention further provides a method for making a wrist support as aforesaid which comprises:

providing a piece of fabric rendered resiliently stretchable by elastomeric yarn woven in one direction, said fabric consisting of a central band of less easily stretchable material bounded by bands of more easily stretchable material, the central band being narrower than one of the outer bands and more than twice the width of the other, the direction of said bands defining a transverse direction of the eventual support;

cutting along the free edge of the wider of the more stretchable bands to conform the free edge to the palm of the hand and form the distal edge of the support;

cutting a concave thumb opening in one lateral edge of the fabric in the wider of the more stretchable bands;

forming the attachment means along the lateral edges of said fabric piece; and forming a brace pocket along the longitudinal direction of the support.

The support is provided on its outer face wth a ventral pocket for insertion of a shaped support of rigid material defining the degree of dorsoflexion of the hand.

Conveniently Velcro (Registered Trade Mark) tabs on one side edge of the support locate onto a Velcro pad on the other side edge to fasten the support about the wrist.

An embodiment of the invention will now be described by way of example only with reference to the accompanying drawings, in which:

Figure 1 is a view of the outer face of a wrist support laid out flat; and

Figure 2 is a view of a brace that fits into the support of Figure 1.

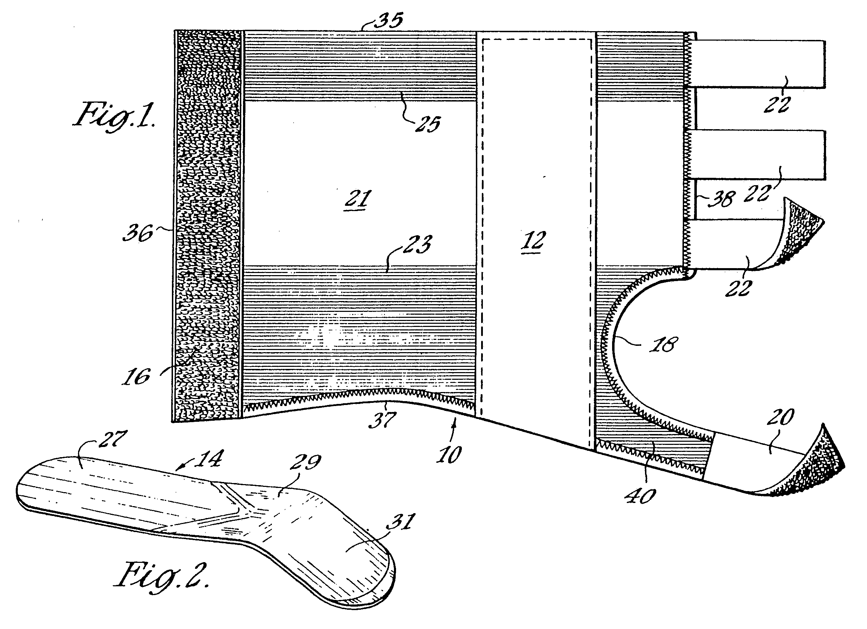

In the drawings, a fabric wrist support 10 is formed on its outer face with a pocket 12 into which may be inserted a metal brace 14 to restrict mobility of the wrist and the palm of the hand. The brace 14 comprises a region 27 which is concave in the transverse direction to underlie and conform to the curvature of the forearm and increase the rigidity of the brace, a planar region 29 that underlies the wrist and an upwardly cranked region 31 that underlies the palm of the hand and holds it in dorsoflexion. Along the outer face of one side edge of the support 10 is attached a Velcro fastening strip 16 of the loop type. The other side edge is concavely profiled at 18 to define a thumb aperture and has a tab 20 that is passed between the thumb and index finger and wrist and forearm tabs 22. The tabs 20, 22 are of hook Velcro material complementary to that of the support 10. The pocket 12 is located so that when the brace 14 is in place and is positioned properly along the palm and forearm the thumb engages in the wrist aperture 18. The Velcro band 16 is located so as to be aligned with the forefinger and to extend along the dorsal surface of the forearm. The edges 36, 38 abut when the support is being worn and the edge 38 is spaced sufficiently widely from the pocket 12 that the brace 14 lies in correct alignment along the ventral surface of the forearm. The peninsular 40 of fabric leading to the tab 20 is made long enough to extend around the forefinger to the band 16. If there is insufficient fabric between edge 38 and pocket 12 and if the peninsular 40 is too short, the thumb aperture provides only a restricted movement of the thumb and the brace 14 is pulled transversely out of alignment with the forearm.

It will be noted that the body of the wrist support is formed from a single piece of fabric having a central wrist region or band 21 of less stretchable material and distal and proximal regions or bands 23, 25 of more stretchable material positioned to correspond to the palm and forearm of the wearer. A suitable fabric for making a wrist support according to the invention has the following characteristics:

| Width | 187 mm Overall |
| | Materials |
| | Cotton 83.046% |
| | Rubber 16.954% |
| | Body 108 Ends×16/2 Cotton |
| | Centre 116 Ends×40/2 Cotton |
| | Cotton covered rubber 115 Ends×CR.241 (M. Wright & Sons) |
| | Weft 1 End×16/2 Cotton |
| | Picks at Loom 25.6 per 1″ (2.5 cms) |
| | Picks Rest 63 per 1″ (2.5 cms) |
| Stretch | Unfinished 120% |
| | Finished 130% |

Spacing of fabric/overall width 187 mm

| 29 mm | 15 Rubbers | Proximal band 25 |
| | 26 Ends 16/2 Cotton | |
| | 12 Rubbers per 1″ (2.5 cms) | |
| 68 mm | 57 Rubbers | Central Band 21 |
| | 116 Ends 40/2 Cotton | |
| | 22 Rubber per 1″ (2.5 cms) | |
| 90 mm | 43 Rubbers | Material to form distal band 23 |
| | 83 Ends 16/2 Cotton | |
| | 12 Rubbers per 1″ (2.5 cms) | |

The wrist region or band 21 is asymmetrically located to provide maximum support to the wrist joint, with the width of the central band 21 being more than twice that of the proximal band 25 of more stretchable material but less than the greatest width of the distal band 23 of more stretchable material. The thumb aperture 18 is formed only in the wider region 20 of more easily stretchable material. The fabric is made resiliently stretchable by the presence of transverse ends of elastomeric material and the central band 21 contains more elastomeric ends per unit length than the proximal or distal bands 25, 23.

The wrist support of the invention is durable and effective. It will be noted that the support of the invention is easy to make because only one piece of stretch fabric is used and that fabric has bands of different elastication of the required widths and characteristics. The proximal edge 35 need not be cut and only a straight cut is needed at the lateral edge 36 that carries the Velcro strip 16. Only the distal edge 37 of the wider more flexible band 23 need be cut to conform, when folded over, to the shape of the palm, and a concave generally semi-circular cut-out 18 is formed in the other lateral edge 38 which also has attached thereto the Velcro tabs 20, 22. Only the minimum of cutting and edge binding operations are required.

**Claims**

1. A wrist support adapted to extend along and surround the forearm, wrist and palm of the hand to restrict mobility of the wrist joint, the support being formed from stretch fabric comprising a combination of more and less easily stretchable transverse bands (21, 23, 25), the support being formed in its outer surface with a longitudinal pocket (12) for a thin brace (14) and having a cut-out defining a thumb aperture (18) positioned relative to the longitudinal pocket (12) so that when in place the pocket (12) extends along the ventral surface of the forearm and wrist and underlies the palm, and the band (21) of less stretchable material being positioned towards the proximal end of the support so as to act on the wrist but substantially not on the palm, characterised in that the support is formed from a single piece of fabric consisting of a single less easily stretchable band (21) between proximal and distal more easily stretchable bands (25, 23) and in that the support has lateral edges with attachment means (16, 20, 22) thereat for fastening the lateral edges together about the wrist.

2. A wrist support according to Claim 1 wherein the distal band (23) of more stretchable material is wider than the proximal band (25) of more stretchable material and a generally semi-circular cut-out defining the thumb aperture (18) is formed only in the distal band (23).

3. A wrist support according to Claim 1 or 2, wherein the fabric is made resiliently stretchable by the presence of transverse ends of elastomeric material and the central band (21) contains more elastomeric ends per unit length than the proximal or distal bands (25, 23).

4. A wrist support according to Claim 3, wherein the width of the central band (21) is more than twice that of the proximal band (25) of more stretchable material but less than the greatest width of the distal band (23) of more stretchable material.

5. A wrist support according to any preceding claim, wherein the brace (14) is fitted in the pocket (12), extends similarly to the pocket (12) and holds the hand in dorsoflexion, and comprises a transversely concave proximal region (27) to underlie and conform to the forearm, a flat region (29) to underlie the wrist and an upwardly cranked region (31) to underlie the palm.

6. A support according to any preceding claim, wherein the fastening means comprises tabs (20, 22) of hook-type fibrous fastening fabric along one lateral edge and a strip (16) of eye-type fibrous fastening fabric along the other edge.

7. A method of making a wrist support according to Claim 1, including the steps of:

providing a piece of fabric rendered resiliently stretchable by elastomeric yarn woven in one direction, said fabric consisting of a central band (21) of less easily stretchable material bounded by bands (25, 23) of more easily stretchable material, the central band (21) being narrower than one of the outer bands (23) and more than twice the width of the other (25), the direction of said bands (21, 23, 25) defining a transverse direction of the eventual support;

cutting along the free edge (37) of the wider of the more stretchable bands (23) to conform the free edge (37) to the palm of the hand and form the distal edge of the support;

cutting a concave thumb opening (18) in one lateral edge (38) of the fabric in the wider of the more stretchable bands (23); and

forming the attachment means (16, 20, 22) along the lateral edges of said fabric piece; and

forming a brace pocket along the longitudinal direction of the support.

**Patentansprüche**

1. Handgelenkstütze, zum Anlegen längs des Unterarms, des Handgelenks und der Handfläche und um dieselben herum, um die Beweglichkeit des Handgelenks zu beschränken, welche Stütze aus dehnbarem Gewebe gebildet ist, das aus einer Kombination von leichter und weniger leicht dehnbaren Querbändern (21, 23, 25) besteht, und welche Stütze an ihrer äusseren Oberfläche mit einer länge verlaufenden Taache (12) für ein dünnes Versteifungselement (14) versehen ist und einen Ausschnitt aufweist, der eine Daumenöffnung (18) bildet, welche bezüglich der längs verlaufenden Tasche (12) so angeordnet ist, dass im angelegten Zustand die Tasche (12) sich entlang der Unterseitenfläche des Unterarms und des Handgelenks erstreckt und unter der Handfläche liegt, wobei das Band (21) aus weniger dehnbarem Material sich im Bereich des körpernahen Endes der Stütze befindet, um auf das Handgelenk zu wirken, jedoch im wesentlichen nicht auf die Handfläche, dadurch gekennzeichnet, dass die Stütze aus einem einzigen Gewebestück gebildet ist, das aus eineim einzigen weniger leicht dehnbaren Band (21) zwischen körpernah und körperfern angeordneten leichter dehnbaren Bändern (25, 23) besteht, und dass die Stütze seitliche Ränder mit daran angebrachten Befestigungsmiteln (16, 20, 22) zum Befestigen der seitlichen Rähnder aneinander um das Handgelenk herum aufweist.

2. Handgelenkstütze nach Anspruch 1, in welcher das körperferne Band (23) aus leichter dehnbarem Material breiter ist als des köpernahe Band (25) aus leichter dehnbarem Material und in welchem ein allgemein halbkreisförmiger Ausschnitt, der die Daumenöffnung (18) bildet, ganz in dem körperfernen Band (23) liegt.

3. Handgelenkstütze nach Anspruch 1 oder 2, in welcher das Gewebe durch die Gegenwart von quer verlaufenden Fäden aus Elastomermaterial elastisch dehnbar ist und das mittlere Band (21) mehr Elastomerfäden pro Längeneinheit enthält alt das körpernahe und das körperferne Band (25, 23).

4. Handgelenkstütze nach Anspruch 3, in welcher die Breite des mittleren Bandes (21) mehr als doppelt so gross ist wie die Breite des körpernahen Bandes (25) aus leichter dehnbarem Material, aber kleiner ist als die grösste Breite des körperfernen Bandes (23) aus leichter dehnbarem Material.

5. Handgelenekstütze nach einem der vorangehenden Ansprüche, in welcher das Versteifungselement (14) in die Tasche (12) eingepasst ist, sich ähnlich wie die Tasche (12) erstreckt und die Hand nach oben zurückgebogen hält, wozu es einen in Querrichtung konkaven körpernahen Abschnitt (27) für die passende Anlage am Unterarm, einen flachen Abschnitt (29) für die Anlage am Handgelenkt und einen nach oben abgekröpften Abschnitt (31) für die Anlage an der Handfläche aufweist.

6. Stütze nach einem der vorangehenden Ansprüche, in welcher die Befestigungsmittel Laschen (20, 22) aus mit Häkchen besetztem Haft-Faserflächenmaterial längs eines der seitlichen Ränder und einen Streifen aus Fläsch-Faserflächenmaterial längs des anderen Randes enthalten.

7. Verfahren zur Herstellung einer Handgelenkstütze nach Anspruch 1, enthaltend die folgenden Schritte:

Zurverfügungstellen eines Gewebestückes, das durch in einer Richtung eingewobenes Elastomergarn elastisch dehnbar gemacht ist und das aus einem mittleren Band (21) aus weniger leicht dehnbarem Material und aus an das mittlere Band angrenzenden Bändern (25, 23) aus leichter dehnbarem Material besteht, wobei das mittlere Band (21) schmaler als eines der äusseren Bänder (23) und mehr als doppelt so breit wie das andere (25) ist und wobei die Richtung der genannten Bänder (21, 23, 25) eine Querrichtung der herzustellenden Stütze definiert;

Schneiden längs des freien Randes (37) des breiteren der beiden leichter dehnbaren Bänder (23) zur Anpassung dieses freien Randes (37) an die Handfläche und Bildung des körperfernen Randes der Stütze;

**0 162 610**

Schneiden einer konkaven Daumenöffnung (18) in einen seitlichen Rand (38) des Gewebes im breiteren der beiden leichter dehnbaren Bänder (23);

Bilden der Befestigungsmittel (16, 20, 22) längs der seitlichen Ränder des Gewebestückes; und

Bilden einer Versteifungselementtasche längs der Längsrichtung der Stütze.

**Revendications**

1. Manchette pour poignet conçue pour s'étendre le long et autour de l'avant-bras, du poignet et de la paume de la main afin de limiter la mobilité de l'articulation du poignet et formée d'un tissu extensible comprenant, en combinaison, des bandes transversales plus et moins facilement extensibles, cette manchette comportant, dans sa surface extérieure, une poche longitudinale destinée à une armature mince et présentant, une découpe définissant une ouverture pour le pouce, dont la position par rapport à la poche longitudinale est telle qu'après mise en place, la poche s'étende le long de la surface ventrale de l'avant-bras et du poignet et sous la paume, la band de matière moins extensible étant située près de l'extrémité proximale de la manchette pour agir sur le poignet, mais en substance pas agir sur la paume.

2. Manchette pour poignet suivant la revendication 1, dans laquelle la bande distale (23) en matière plus extensible est plus large que la bande proximale (25) en matière plus extensible, et une découpe dans l'ensemble semi-circulaire définissant l'ouverture (18) pour le pouce n'est ménagée que dans la bande distale (23).

3. Manchette pour poignet suivant la revendication 1 ou 2, dans laquelle le tissu est rendu élastiquement extensible par la présence de brins transversaux en matière élastomère et la bande médiane (21) contient un plus grand nombre de brins élastomères par longueur unitaire que les bandes proximale et distale (25, 23).

4. Manchette pour poignet suivant la revendication 3, dans laquelle la largeur de la bande médiane (21) est égale à plus du double de celle de la bande proximale (25) en matière plus extensible, mais est inférieure à la plus grande largeur de la bande distale (23) en matière plus extensible.

5. Manchette pour poignet suivant l'une quelconque des revendications précédentes, dans laquelle l'armature (14) est ajustée dans la poche (12), s'étend de la même manière que la poche (12) et maintient la main en flexion dorsale, et elle comprend une région proximale concave dans le sans transversal pour s'adapter sous l'avant-bras en épousant la forme de celui-ci, une région plane (29) s'adaptant sous le poignet et une région coudée vers le haut (31) s'adaptant sous la paume.

6. Manchette pour poignet suivant l'une quelconque des revendications précédentes, dans laquelle les moyens d'attache comprennent des pattes (20, 22) de tissu de fixation fibreux de type à crochets le long d'un bord latéral et une bande (16) de tissu de fixation fibreux du type à bouclettes le long de l'autre bord.

7. Procédé de confection d'une manchette pour poignet suivant la revendication 1, suivant lequel on prévoit une pièce de tissue rendue élastiquement extensible par un fil élastomère tissé dans une direction, ce tissu étant constitué d'une bande médiane de matière moins facilement extensible bornée de bandes de matière plus facilement extensible, la bande médiane étant plus étroite que l'une des bandes extérieures et présentant une largeur égale à plus du double de celle de l'autre, le sens des dites bandes définissant le sens transversale de la manchette finale,

on découpe la pièce le long due bord libre de la plus large des bandes plus facilement extensibles de manière que le bord libre épouse de la forme de la paume de la main et forme le bord distal de la manchette,

on découpe une ouverture concave pour le pouce dans un bord latéral du tissu dans la plus large des bandes plus facilement extensibles (23),

on forme les moyens d'attache le long des bords latéraux de la dite pièce de tissu, et

on forme une poche pour armature dans le sens longitudinal de la manchette.

Fig.1.

Fig.2.

0 162 610